(19) Europäisches Patentamt
European Patent Office
Office européen des brevets

(11) **EP 4 781 932 A1**

(12) **EUROPÄISCHE PATENTANMELDUNG**

(43) Veröffentlichungstag:
**29.07.2026 Patentblatt 2026/31**

(21) Anmeldenummer: **25154540.6**

(22) Anmeldetag: **28.01.2025**

(51) Internationale Patentklassifikation (IPC):
**A61B 18/12** $^{(2006.01)}$     **A61B 18/00** $^{(2006.01)}$

(52) Gemeinsame Patentklassifikation (CPC):
**A61B 18/1206; A61B 18/1233; H02M 1/0019; H02M 1/0025;** A61B 18/14; A61B 90/98; A61B 2018/00178; A61B 2018/00648; A61B 2018/00684; A61B 2018/00767; A61B 2018/00827; A61B 2018/00875; A61B 2018/00892; A61B 2018/00988; A61B 2018/1253;     (Forts.)

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(71) Anmelder: **Erbe Elektromedizin GmbH**
**72072 Tübingen (DE)**

(72) Erfinder:
• **WEILER, Rolf**
**72074 Tuebingen (DE)**
• **MAIER, Phillipp**
**72072 Tuebingen (DE)**

(74) Vertreter: **Rüger Abel Patentanwälte PartGmbB**
**Webergasse 3**
**73728 Esslingen a. N. (DE)**

(54) **VERSORGUNGSGERÄT ZUR VERSORGUNG EINES ELEKTROMEDIZINISCHEN INSTRUMENTS**

(57) Die Erfindung betrifft ein Versorgungsgerät (11) zur Versorgung eines elektromedizinischen Instruments (12) mit elektrischer Energie. Ein einstellbarer und/oder auswählbarer Betriebsmodusparameter (M) definiert eine Betriebsmodusspannung ($U_M$), die einen Sollwert für eine Elektrodenspannung ($U_E$) an einer Elektrode (15, 16) des elektromedizinischen Instruments (12) angibt. An einem Netzteilausgang (24) eines Netzteils (23) des Versorgungsgeräts (11) wird eine eingeprägte Ausgangsspannung ($U_A$) entsprechend einer Ausgangssollspannung ($U_S$) sowie ein variabler Ausgangsstrom ($I_A$) für das elektromedizinische Instrument (12) bereitgestellt. Aufgrund von der Impedanz des Instruments (12) kann sich eine Blindspannung im elektrischen Pfad zu der Elektrode (15, 16) bilden, so dass die Elektrodenspannung ($U_E$) nicht der gewünschten Betriebsmodusspannung ($U_M$) entspricht. Deswegen wird die Betriebsmodusspannung basierend auf einem Korrekturwert (C) und insbesondere einer Korrekturspannung ($U_C$) korrigiert und bildet eine korrigierte Betriebsmodusspannung ($U_{MC}$), basierend auf der die Ausgangssollspannung ($U_S$) für das Netzteil (23) vorgegeben wird. Der Korrekturwert (C) bzw. die Korrekturspannung ($U_C$) werden abhängig vom Istwert des Ausgangsstromes ($I_A$) sowie einem Impedanzparameter (ZI) ermittelt, der die Impedanz des Instruments (12) kennzeichnet.

Fig. 5

EP 4 781 932 A1

(52) Gemeinsame Patentklassifikation (CPC): (Forts.)
A61B 2018/126; H02M 3/33507

**Beschreibung**

Die Erfindung betrifft ein Versorgungsgerät für ein elektromedizinisches Instrument und insbesondere ein elektrochirurgisches Instrument. Das Versorgungsgerät ist dazu eingerichtet, ein daran angeschlossenes elektromedizinisches Instrument mit elektrischer Energie zu versorgen, insbesondere um elektrische Leistung für wenigstens eine Elektrode des Instruments bereitzustellen. Die Erfindung betrifft auch ein Verfahren um ein elektromedizinisches Instrument mit elektrischer Energie zu versorgen sowie ein System aufweisend ein Versorgungsgerät und ein daran angeschlossenes elektromedizinisches Instrument.

DE 10 2015 212 359 A1 offenbart ein Hochfrequenz-Elektrochirurgie-Instrument, das mittels einem Stecker und einem Kabel elektrisch an einen Hochfrequenzgenerator angeschlossen werden kann. Der Hochfrequenzgenerator kann dazu eingerichtet sein, bei einem erkannten Kurzschluss zwischen zwei Elektroden des Instruments die elektrische Versorgung des Instruments abzuschalten. Um eine Kurzschlussermittlung nicht durch den Widerstand des Kabels bzw. des elektrochirurgischen Instruments zu beeinträchtigen, weist das Instrument eine Anpassungsschaltung zur Impedanzanpassung auf. Mittels der Anpassungsschaltung sollen parasitäre Kapazitäten und/oder Induktivitäten des Kabels und des Instruments kompensiert werden. Die Anpassungsschaltung kann auch eine Abstimmspule aufweisen, um eine Resonanzfrequenz des Hochfrequenz-Elektrochirurgie-Instruments auf eine gewünschte Arbeitsfrequenz des Hochfrequenzgenerators abzustimmen.

Eine solche Anpassungsschaltung muss allerdings für jedes Instrument und gegebenenfalls für jede Anwendungsart eines Instruments bereitgestellt werden. Dadurch werden Instrumente teuer und aufwendig, was insbesondere bei Einweginstrumenten unerwünscht ist.

DE 689 118 67 T2 betrifft ein Verfahren und ein Versorgungsgerät zur Versorgung einer Patientenelektrode mit elektrischer Leistung. Ein Leistungsverstärker, an den die Patientenelektrode angeschlossen ist, erzeugt Ausgangsmesssignale, die der Ausgangsspannung und dem Ausgangsstrom entsprechen. Aus diesen Messwerten wird die aktuelle Lastimpedanz am Ausgang des Leistungsverstärkers berechnet und abhängig davon durch eine Kurve bzw. Kurvenschar wird die am Ausgang des Leistungsverstärkers bereitzustellende elektrische Leistung bestimmt und entsprechend geregelt. Wenn die Impedanz allerdings durch parasitäre Effekte oder ähnliches beeinträchtigt wird, kann dies zu einer falschen Zuordnung eines einzustellenden Leistungswertes führen.

Unterschiedliche elektromedizinische Instrumente und unterschiedliche Anwendungsarten eines elektromedizinischen Instruments können dazu führen, dass sich eine Impedanz am Ausgang eines Versorgungsgerätes ändert, an den das elektromedizinische Instrument angeschlossen ist. Die sich abhängig vom Instrument und/oder dessen Betriebsart ändernde Impedanz kann wiederum dazu führen, dass an einer Elektrode des elektromedizinischen Instruments nicht die in einer Steuerung oder Regelung des Versorgungsgerätes vorgegebene Sollspannung zur Verfügung steht und es daher zu unerwünschten Spannungsabweichungen kommt. Solche Spannungsabweichungen können nachteilig bei der Behandlung biologischen Gewebes sein.

Es ist daher die Aufgabe der vorliegenden Erfindung, unerwünschte Spannungsabweichungen in einem elektromedizinischen Instrument, insbesondere an wenigstens einer zur Behandlung von Gewebe dienenden Elektrode, bei dessen Versorgung mit elektrischer Leistung bzw. Energie zu reduzieren oder zu vermeiden.

Diese Aufgabe wird mittels eines Versorgungsgerätes mit den Merkmalen des Patentanspruches 1, einem System mit den Merkmalen des Patentanspruches 10 sowie einem Verfahren mit den Merkmalen des Patentanspruches 14 gelöst.

Das erfindungsgemäße Versorgungsgerät ist zur Versorgung eines elektromedizinischen Instruments mit elektrischer Leistung bzw. Energie eingerichtet. Das Versorgungsgerät hat ein Netzteil, das an seinem Netzteilausgang eine eingeprägte Ausgangsspannung bereitstellt. Die Ausgangsspannung wird vorzugsweise durch einen Netzteilregler entsprechend einer vorgegebenen Ausgangssollspannung geregelt. Am Netzteilausgang stellt das Netzteil einen Ausgangsstrom bereit, der lastabhängig variieren kann.

Die Impedanz des an das Versorgungsgerät angeschlossenen elektromedizinischen Instruments kann variieren. Beispielsweise weisen elektromedizinische Instrumente unterschiedlichen Typs, unterschiedliche Impedanzen auf. Außerdem können auch elektromedizinische Instrumente gleichen Typs durch toleranzbehaftete Komponenten unterschiedliche Impedanzen aufweisen. Solche Toleranzen und Schwankungen bei der Impedanz führen zu Abweichungen einer zur Behandlung von biologischem Gewebe anliegenden Spannung, insbesondere einer Elektrodenspannung an einer zur Behandlung von biologischem Gewebe eingerichteten Elektrode des Instruments. Diese Elektrode kann an einem distalen Ende des Instruments angeordnet sein. Beispielsweise kann eine Spannungsabweichung auf einem durch einen nicht eingeprägten Ausgangsstrom und die Impedanz bewirkten Blindspannungsanteil beruhen. Im Instrument, beispielsweise an der Elektrode des Instruments, über die das Gerät und das biologische Gewebe miteinander verbunden sind, steht dann nicht mehr die gewünschte Spannung (z.B. Elektrodenspannung) zur Verfügung.

Um dies zu vermeiden, weist das Versorgungsgerät eine Sollwerteinstelleinrichtung auf. Die Sollwerteinstelleinrichtung ist dazu eingerichtet, die Ausgangssollspannung derart zu ermitteln, dass ein durch die Impedanz des elektromedizinischen Instruments verursachter Blindspannungsanteil ganz oder teilweise kompensiert wird.

Die Sollwerteinstelleinrichtung hat hierfür eine Ermittlungseinheit sowie eine Korrektureinheit. Die Korrektureinheit ist dazu eingerichtet, einen Korrekturwert zu ermitteln, der abhängig ist von einem aktuellen Istwert eines Ausgangsstromes am Ausgang des Versorgungsgeräts und abhängig ist von wenigstens einem Impedanzparameter. Der Impedanzparameter charakterisiert die elektrische Impedanz des zu versorgenden elektromedizinischen Instruments. Der Impedanzparameter kann als Innenwiderstand einer Spannungsquelle betrachtet werden. Der Impedanzparameter kann beispielsweise für einen Instrumententyp oder für jedes elektromedizinische Instrument individuell durch eine Simulation und/oder Messung ermittelt und abgespeichert werden. Der Impedanzparameter kann entweder in einem Speicher des elektrochirurgischen Instruments und/oder in einem Speicher des Versorgungsgeräts und/oder in einem externen Speicher (Cloud-Speicher) abgelegt werden. Jedenfalls wird der Impedanzparameter einem Instrumententyp oder einem individuellen elektromedizinischen Instrument zugeordnet und steht der Korrektureinheit zur Verfügung.

Als der wenigstens eine Impedanzparameter kann ein wenigstens ein Impedanzwert verwendet werden. Zusätzlich oder alternativ kann der Impedanzparameter auch eine Ersatzschaltung bzw. ein Ersatzschaltbild (z.B. komplexes Ersatzschaltbild mit Real- und Imaginärteil), eine Funktion, eine Kennlinie, ein Kennfeld oder ähnliches sein. Der wenigstens eine Impedanzparameter kann wiederum abhängig sein von wenigstens einem weiteren Parameter, wie zum Beispiel einer Frequenz der Ausgangsspannung und/oder des Ausgangsstromes.

Der Korrekturwert wird dazu verwendet, eine für einen aktuellen Betriebsmodus des Versorgungsgerätes voreingestellte Betriebsmodusspannung zu korrigieren und daraus die Ausgangssollspannung zu ermitteln, die dann dem Netzteil bereitgestellt wird. Das Netzteil erzeugt somit eine Ausgangsspannung, die abhängig vom Impedanzparameter des zu versorgenden elektromedizinischen Instruments korrigiert ist. Dadurch kann im Instrument, insbesondere an einer Elektrode des Instruments, die gewünschte Instrumentenspannung bzw. Elektrodenspannung mit nur geringer oder keiner Abweichung anliegen. Eine an der Impedanz im Instrument anliegende und daher vom Impedanzparameter abhängige Spannung muss dabei nicht gemessen werden.

Insbesondere kann über eine Bedienerschnittstelle oder eine andere Einrichtung des Versorgungsgerätes ein Betriebsmodusparameter ausgewählt und eingestellt werden. Der Betriebsmodusparameter definiert die Betriebsmodusspannung oder ist die Betriebsmodusspannung, die für den gewählten Betriebsmodus des Versorgungsgerätes als Instrumentenspannung bzw. Elektrodenspannung bereitgestellt werden soll. Der Korrekturwert C definiert eine Korrekturspannung oder ist eine Korrekturspannung, wobei die Betriebsmodusspannung basierend auf der Korrekturspannung mittels der Sollwerteinstelleinrichtung korrigiert wird, so dass eine korrigierte Betriebsmodusspannung gebildet wird, deren Betrag insbesondere größer ist als der Betrag der Betriebsmodusspannung. Bei einem bevorzugten Ausführungsbeispiel kann die korrigierte Betriebsmodusspannung die Summe aus der Betriebsmodusspannung und der Korrekturspannung sein. Alternativ hierzu kann die Betriebsmodusspannung auch über einen Korrekturfaktor korrigiert werden, der in diesem Fall den Korrekturwert darstellt.

Der Korrekturwert und beispielsweise die Korrekturspannung kann basierend auf einer Funktion oder einem Algorithmus ermittelt werden, der als Eingangsparameter den Istwert des Ausgangsstromes und den Impedanzparameter verwendet.

Dadurch, dass der Impedanzparameter des elektromedizinischen Instruments ermittelt wird und der Ausgangsstrom beim Betrieb erfasst und der Korrektureinheit bereitgestellt wird, kann die über den Betriebsmodusparameter ausgewählte Betriebsmodusspannung während des Betriebs des Versorgungsgerätes, also während der Verwendung eines daran angeschlossenen elektromedizinischen Instruments, stets angepasst werden, so dass im elektromedizinischen Instrument die gewünschte Instrumentenspannung bzw. Elektrodenspannung bereitsteht.

Bei einem Ausführungsbeispiel kann der Impedanzparameter für ein oder mehrere elektromedizinische Instrumente, die an das Versorgungsgerät anschließbar sind, in der Korrektureinheit gespeichert sein, wobei der Impedanzparameter veränderbar oder unveränderbar sein kann.

Bei einem Ausführungsbeispiel des Versorgungsgeräts ist es dazu eingerichtet, einen Kennwert eines an das Versorgungsgerät angeschlossenen elektromedizinischen Instruments zu ermitteln und beispielsweise aus einem Speicher des elektromedizinischen Instruments auszulesen. Der Kennwert kann das individuelle elektromedizinische Instrument und/oder den Instrumententyp des elektromedizinischen Instruments charakterisieren. Anhand des Kennwerts kann im Versorgungsgerät und insbesondere mittels der Korrektureinheit der zu dem angeschlossenen elektromedizinischen Instrument gehörende Impedanzparameter ermittelt werden. Im einfachsten Fall kann der Kennwert bereits den Impedanzparameter aufweisen oder der Kennwert ist der Impedanzparameter.

Es kann vorteilhaft sein, die elektromedizinischen Instrumente nach deren Herstellung zu testen und deren individuelle Impedanz zu ermitteln und in einem Speicher des elektromedizinischen Instruments abzuspeichern, so dass der Impedanzparameter durch das Versorgungsgerät ausgelesen werden kann. Alternativ kann es auch zweckmäßig sein, mittels einer Tabelle oder einer anderen Zuordnungsvorschrift einem dem Versorgungsgerät bereitgestellten Kennwert des angeschlossenen elektromedizinischen Instruments mittels der Korrektureinheit einem Impedanzparameter zuzuordnen.

Ein elektromedizinisches Instrument kann einen einzigen Impedanzparameter oder mehrere Impedanzparameter aufweisen. Beispielsweise kann unterschiedlichen Anwendungsarten oder Betriebsarten eines elektromedizinischen Instruments jeweils ein Impedanzparameter zugeordnet sein. Ein elektromedizinisches Instrument kann zum Beispiel bei einem Schneidbetrieb einen anderen Impedanzparameter aufweisen als bei einem Koagulationsbetrieb. Es ist daher vorteilhaft, wenn der Impedanzparameter abhängig von der aktuellen Betriebsart bzw. dem aktuellen Betriebsmodus ermittelt wird. Der Betriebsmodus kann beispielsweise anhand des Betriebsmodusparameters erkannt werden.

Irgendein Ausführungsbeispiel des Versorgungsgeräts, wie es vorstehend erläutert wurde, kann Bestandteil eines Systems sein, das außerdem ein an das Versorgungsgerät angeschlossenes elektromedizinisches Instrument aufweist. Das elektromedizinische Instrument ist vorzugsweise ein elektrochirurgisches Instrument.

Bei verschiedenen Ausführungsbeispielen kann das elektromedizinische Instrument ein monopolares Instrument oder ein bipolares Instrument sein. Im Falle eines bipolaren Instruments wird die Elektrodenspannung vorzugsweise zwischen zwei Elektroden des Instruments ermittelt. Im Falle eines monopolaren Instruments kann eine separate neutrale Elektrode vorhanden sein, die zum Anbringen an den Patienten eingerichtet ist. Die Elektrodenspannung kann dann zwischen der Elektrode des monopolaren Instruments und der separaten Neutralelektrode ermittelt werden. In beiden Fällen ist die durch den Betriebsmodusparameter definierte Betriebsmodusspannung ein Sollwert für die Elektrodenspannung. Das Instrument kann demnach wenigstens eine, zwei oder auch mehr als zwei Elektroden aufweisen, beispielsweise wenn das elektromedizinische Instrument für unterschiedliche Anwendungsarten eingerichtet ist, wie etwa zum Schneiden von Gewebe, als auch für das Koagulieren von Gewebe.

Das elektromedizinische Instrument hat vorzugsweise einen Instrumentenschaltkreis, wobei der Instrumentenschaltkreis zumindest ein elektrisches und/oder elektronisches Bauelement enthält. Der Instrumentenschaltkreis umfasst insbesondere ein kapazitives Bauelement und/oder ein induktives Bauelement. Beispielsweise kann der Instrumentenschaltkreis einen Koppelkondensator aufweisen, an den die Elektrode oder eine der vorhandenen Elektroden angeschlossen ist, vorzugsweise unmittelbar. Zusätzlich oder alternativ kann der Instrumentenschaltkreis auch einen Transformator, beispielsweise einen Spartransformator, und/steuerbare Bauelemente und/oder schaltbare Bauelemente aufweisen.

Unter Verwendung irgendeines Ausführungsbeispiels eines Versorgungsgerätes oder eines Systems, wie es vorstehend beschrieben wurde, kann ein erfindungsgemäßes Verfahren durchgeführt werden.

Das erfindungsgemäße Verfahren ist zur Versorgung eines elektromedizinischen Instruments eingerichtet. Zunächst wird an einem Netzteilausgang eines Netzteils ein lastabhängig variabler Ausgangsstrom sowie eine eingeprägte Ausgangsspannung bereitgestellt. Die eingeprägte Ausgangsspannung kann beispielsweise mittels eines Netzteilreglers entsprechend einer vorgegebenen Ausgangssollspannung geregelt werden.

Abhängig von einem Impedanzparameter des zu versorgenden elektromedizinischen Instruments und abhängig von einem aktuellen Istwert des Ausgangsstromes wird ein Korrekturwert ermittelt, vorzugsweise unter Verwendung einer Funktion oder eines Algorithmus. Der Korrekturwert wird gemeinsam mit einem Betriebsmodusparameter verwendet, um daraus die Ausgangssollspannung für das Netzteil zu ermitteln. Insbesondere kann der Betriebsmodusparameter eine Betriebsmodusspannung definieren, die wiederum den Sollwert einer Instrumentenspannung bzw. Elektrodenspannung im zu versorgenden elektromedizinischen Instrument darstellt. Um Abweichungen der Instrumentenoder Elektrodenspannung von der Betriebsmodusspannung zu reduzieren oder zu eliminieren, wird der Korrekturwert impedanzabhängig und insbesondere auch ausgangsstromabhängig ermittelt und zur Korrektur der durch den Betriebsmodusparameter definierten Betriebsmodusspannung verwendet.

Vorteilhafte Ausgestaltungen der Erfindung ergeben sich aus den abhängigen Ansprüchen, der Beschreibung und der Zeichnung. Nachfolgend werden bevorzugte Ausführungsbeispiele der Erfindung basierend auf der beigefügten Zeichnung im Einzelnen erläutert. In der Zeichnung zeigen:

Figur 1 ein Ausführungsbeispiel eines Systems aufweisend ein Versorgungsgerät und ein daran angeschlossenes bipolares elektromedizinisches Instrument,

Figur 2 ein Ausführungsbeispiel eines Systems aufweisend ein Versorgungsgerät und ein daran angeschlossenes monopolares elektromedizinisches Instrument und

Figur 3 ein Blockschaltbild eines Ausführungsbeispiels eines Versorgungsgeräts sowie eines elektromedizinischen Instruments,

Figur 4 ein Blockschaltbild eines weiteren Ausführungsbeispiels eines Versorgungsgeräts und eines elektromedizinischen Instruments,

Figur 5 ein Blockschaltbild eines Ausführungsbeispiels zur erfindungsgemäßen Ermittlung einer Ausgangssollspannung bei der vorliegenden Erfindung,

Figur 6 ein Blockschaltbild eines Ausführungsbeispiels zur Ermittlung einer Ausgangssollspannung bei der vorliegenden Erfindung und

Figur 7 einen beispielhaften Verlauf eines Stromes und einer Elektrodenspannung an einer Elektrode eines elektromedizinischen Instruments bei der Verwendung der vorliegenden Erfindung.

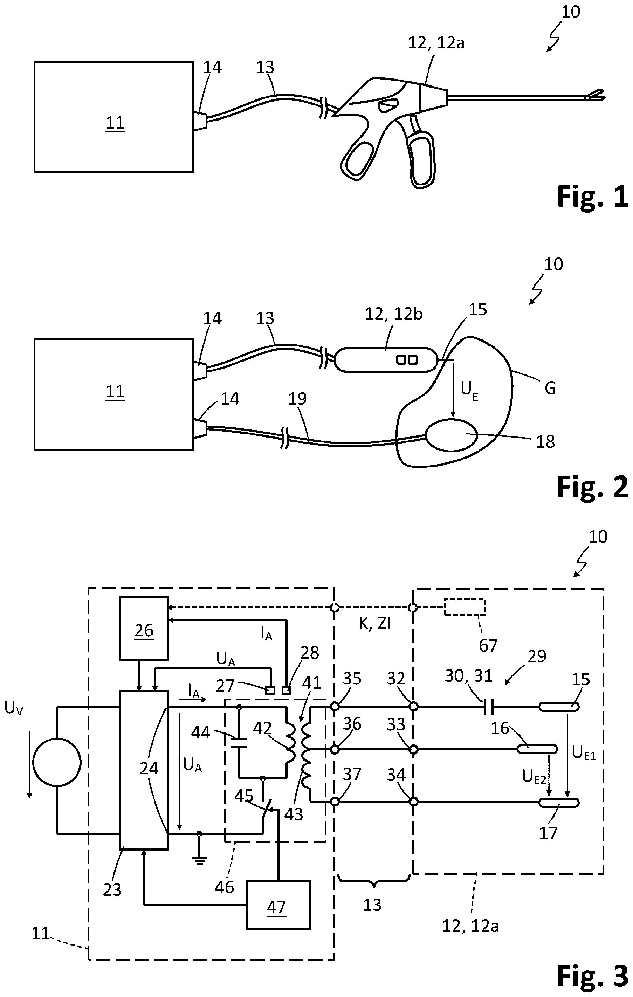

In den Figuren 1 und 2 sind unterschiedliche Ausführungsbeispiele eines Systems 10 schematisch dargestellt. Das System 10 weist ein Versorgungsgerät 11 sowie ein an das Versorgungsgerät 11 angeschlossenes oder anschließbares elektromedizinisches Instrument 12 auf. Das elektromedizinische Instrument 12 kann mit einem Kabel 13 und einem Stecker 14 an das Versorgungsgerät 11 angeschlossen werden. In verbundenem Zustand versorgt das Versorgungsgerät 11 das elektromedizinische Instrument 12 mit elektrischer Energie beziehungsweise elektrischer Leistung. Optional können über das Versorgungsgerät 11 auch andere Medien zum Betrieb des elektromedizinischen Instruments 12 bereitgestellt werden, wie zum Beispiel eine Flüssigkeit oder ein Gas.

Bei den hier veranschaulichten Ausführungsbeispielen handelt es sich bei dem elektromedizinischen Instrument 12 um ein elektrochirurgisches Instrument zum Koagulieren und/oder Schneiden von zu behandelndem Gewebe G, das nachfolgend kurz als Instrument 12 bezeichnet wird.

Das Instrument 12 kann beispielsgemäß als bipolares Instrument 12a (Figur 1) oder als monopolares Instrument 12b (Figur 2) ausgebildet sein. Das bipolare Instrument 12a hat zwei oder mehr Elektroden und beim Ausführungsbeispiel eine erste Elektrode 15, eine zweite Elektrode 16 sowie eine dritte Elektrode 17. Die erste Elektrode 15 kann unter Zusammenarbeit mit der dritten Elektrode 17 zum Koagulieren von Gewebe verwendet werden. Die zweite Elektrode 16 kann beispielsweise mit der dritten Elektrode 17 zusammenarbeiten, um Gewebe zu schneiden. Andere Anwendungen und eine andere Anzahl von Elektroden kann abhängig vom Typ des Instruments 12 gewählt werden.

Das monopolare Instrument 12b hat lediglich eine einzige Elektrode 15. Während bei dem bipolaren Instrument 12a ein Behandlungsstromkreis über die erste Elektrode 15 oder die zweite Elektrode 16 durch das zu behandelnde Gewebe und weiter über die dritte Elektrode 17 zurück zum Versorgungsgerät 11 geschlossen werden kann, ist bei dem monopolaren Instrument 12b zusätzlich eine Neutralelektrode 18 vorhanden, die über ein Elektrodenkabel 19 und einen Stecker 14 mit dem Versorgungsgerät 11 verbunden werden kann. Die Neutralelektrode 18 wird am Patienten in elektrisch leitender Verbindung mit dem zu behandelnden Gewebe G angebracht, wie es stark schematisiert in Figur 2 gezeigt ist. Typischerweise wird die Neutralelektrode 18 außen auf der Haut des Patienten befestigt.

Ein Instrument mit mehreren Polen (bipolares oder multipolares Instrument) kann optional - analog zu einem monopolaren Instrument - auch in monopolarer Betriebsart mit einer der vorhandenen Elektroden betrieben werden. In dieser Betriebsart wird dann zusätzlich die Neutralelektrode 18 verwendet.

Im Folgenden werden Ausführungsbeispiele des Systems 10 bzw. des elektromedizinischen Instruments 12 anhand eines bipolaren Instruments 12a erläutert. Die Erläuterungen können analog auf ein monopolares Instrument 12b mit einer einzigen Elektrode 15 oder ein in monopolarer Betriebsart eingesetztes bi- oder multipolares Instrument 12b übertragen werden, wobei die Funktion der Bezugselektrode nicht von der dritten Elektrode 17, sondern von der Neutralelektrode 18 übernommen wird.

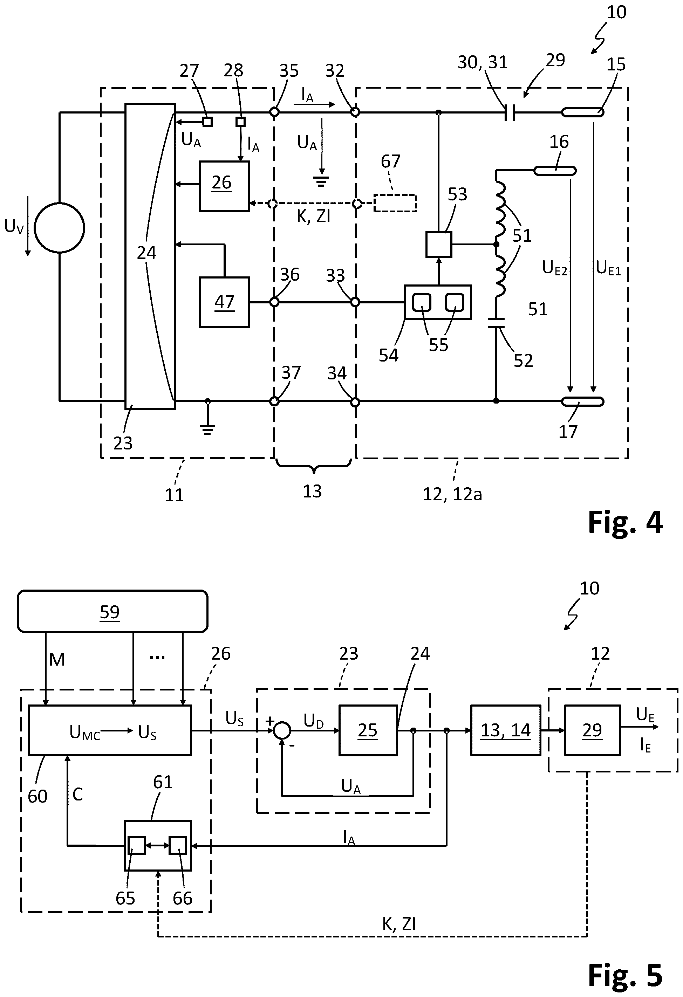

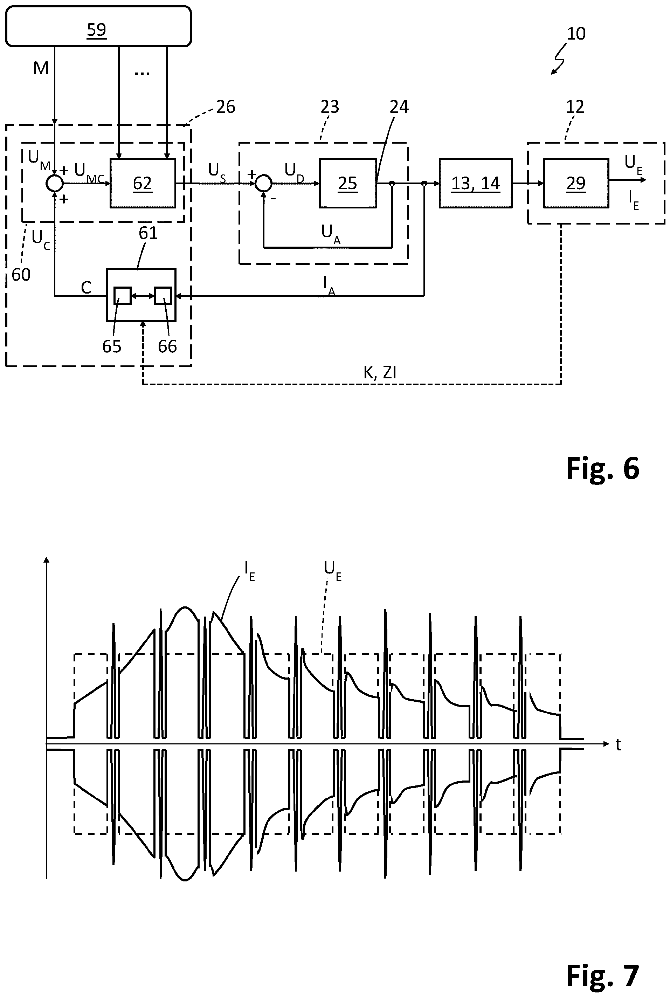

Ein Ausführungsbeispiel eines Systems 10 in Form von einem Schaltplan bzw. einem Blockschaltbild ist in Figur 3 veranschaulicht. Das Versorgungsgerät 11 ist an eine Energieversorgungsquelle anschließbar, die eine Versorgungsspannung $U_V$ bereitstellt. Die Versorgungsspannung $U_V$ ist insbesondere die Netzspannung eines Energieversorgungsnetzes. Die Versorgungsspannung $U_V$ wird einem Netzteil 23 des Versorgungsgeräts 11 bereitgestellt, das dazu eingerichtet ist, an einem Netzteilausgang 24 einen variablen Ausgangsstrom $I_A$ und eine eingeprägte Ausgangsspannung $U_A$ bereitzustellen. Die Ausgangsspannung $U_A$ kann beispielsweise mithilfe eines Netzteilreglers 25 (Figuren 5 und 6) in einem geschlossenen Regelkreis geregelt werden, so dass sie einer vorgegebenen Ausgangssollspannung $U_S$ entspricht.

Zur Ermittlung der Ausgangssollspannung $U_S$ hat das Versorgungsgerät 11 eine Sollwerteinstelleinrichtung 26, die den ermittelten Ausgangsspannungssollwert $U_S$ für das Netzteil 23 bereitstellt.

Die Ausgangsspannung UA wird über einen Spannungssensor 27 erfasst und dem Netzteil 23 bereitgestellt. Mittels eines Stromsensors 28 wird der Ausgangsstrom $I_A$ erfasst und der Sollwerteinstelleinrichtung 26 bereitgestellt. Die Sollwerteinstelleinrichtung 26 ist dazu eingerichtet, den Ausgangsspannungssollwert $U_S$ abhängig vom aktuellen Istwert des Ausgangsstromes $I_A$ zu bestimmen und dem Netzteil 23 vorzugeben.

Der Sollwerteinstelleinrichtung 26 wird zudem ein Impedanzparameter ZI bereitgestellt oder der Impedanzparameter ZI wird abhängig von einem anderen Parameter, insbesondere einem Kennwert K ermittelt. Der Impedanzparameter ZI kennzeichnet die Impedanz des zu versorgenden bzw. an das Versorgungsgerät 11 angeschlossenen Instruments 12. Die Impedanz wird insbesondere durch einen Instrumentenschaltkreis 29 des Instruments 12 bestimmt, der wenigstens ein kapazitives und/oder induktives Bauelement 30 aufweisen. Bei dem in Figur 3 dargestellten Ausführungsbeispiel hat der Instrumentenschaltkreis 29 nur ein einziges Bauelement 30, nämlich einen Koppelkondensator 31. Mit dem Koppelkondensator 31 ist eine der Elektroden verbunden, beispielsgemäß die erste Elektrode 15. In Abwandlung zu dem Ausführungsbeispiel nach Figur 3 kann der Instrumentenschaltkreis 29 andere oder zusätzliche elektrische und/oder elektronische Bauteile aufweisen, beispielsweise einen Transformator, Halbleiterschalter, von einer Bedienperson bedienbare Schalter oder Taster, usw. Der Instrumentenschaltkreis 29 gemäß Figur 3 stellt daher eine sehr einfache Realisierungsform nur mit einem Koppelkondensator 31 dar.

Die erste Elektrode 15 ist bei dem in Figur 3 dargestellten Ausführungsbeispiel über den Koppelkondensator 31 mit einem ersten Instrumenteneingang 32 elektrisch verbunden. Die zweite Elektrode 16 ist mit einem zweiten Instrumenteneingang 33 und die dritte Elektrode 17 mit einem dritten Instrumenteneingang 34 elektrisch verbunden. Über das Kabel 13 sind die Instrumenteneingänge 32, 33, 34 mit jeweils einem zugeordneten Geräteausgang, beispielsgemäß einem ersten Geräteausgang 35, einem zweiten Geräteausgang 36 und einem dritten Geräteausgang 37 des Versorgungsgeräts 11 elektrisch verbunden.

Die im Instrument 12 anliegende Instrumentenspannung ist bei den Ausführungsbeispielen die Elektrodenspannung $U_E$. Die Elektrodenspannung $U_E$ wird zwischen einer aktiven Elektrode (beispielsweise erste Elektrode 15 und/oder zweite Elektrode 16) einerseits und einer Bezugselektrode (dritte Elektrode 17 oder Neutralelektrode 18) andererseits ermittelt.

Eine Elektrodenspannung $U_E$ zwischen der ersten Elektrode 15 und der dritten Elektrode 17 kann als erste Elektrodenspannung $U_{E1}$ und eine Elektrodenspannung $U_E$ zwischen der zweiten Elektrode 16 und der dritten Elektrode 17 als zweite Elektrodenspannung $U_{E2}$ bezeichnet werden.

Das Versorgungsgerät 11 weist bei dem in Figur 3 dargestellten Ausführungsbeispiel einen Transformator 41 mit einer Primärwicklung 42 und einer Sekundärwicklung 43 auf. Die Sekundärwicklung 42 ist mit einem Anschluss an den ersten Geräteausgang 35 und mit dem Anschluss am entgegengesetzten Ende mit dem dritten Geräteausgang 37 elektrisch verbunden. Ein Mittenabgriff der Sekundärwicklung 42 ist bei dieser Ausführungsform elektrisch mit dem zweiten Geräteausgang 36 verbunden. Parallel zur Primärwicklung 42 ist ein Kondensator 44 geschaltet. In Reihe zum Kondensator 44 und der Primärwicklung 42 ist ein gesteuerter Schalter 45 angeordnet. Transformator 41, der Kondensator 44 und der gesteuerte Schalter 45 bilden eine Ausgangsstufe 46, an die der Netzteilausgang 24 angeschlossen ist. Mittels der Ausgangsstufe 46 können Hochspannungsimpulse erzeugt werden, die dem Instrument 12 bereitgestellt beziehungsweise an die Elektroden 15, 16, 17 angelegt werden können. Hierzu kann der gesteuerte Schalter 45 durch eine Steuereinrichtung 47 des Versorgungsgeräts 11 gesteuert werden, insbesondere um Hochspannungsimpulse entsprechend einem vorgegebenen Betriebsmodus des Versorgungsgeräts 11 zu erzeugen und bereitzustellen.

In Abwandlung zu der Darstellung aus Figur 3 können der erste Geräteausgang 35 und der zweite Geräteausgang 36 auch mit zwei individuellen Sekundärwicklungen und/oder Transformatoren verbunden sein, um die Elektrodenspannungen $U_{E1}$ und $U_{E2}$ zu erzeugen.

In Figur 4 ist ein Schaltplan beziehungsweise ein Blockschaltbild eines weiteren Ausführungsbeispiels eines Versorgungsgeräts 11 sowie eines Instruments 12 veranschaulicht. In Abwandlung zum Ausführungsbeispiel nach Figur 3 ist bei dem Versorgungsgerät 11 gemäß Figur 4 der Netzteilausgang 24 mit dem Geräteausgang und beispielsgemäß dem ersten Geräteausgang 35 und dem dritten Geräteausgang 37 verbunden, so dass die Ausgangsspannung $U_A$ zwischen dem ersten Geräteausgang 35 und dem dritten Geräteausgang 37 anliegt. Vom Versorgungsgerät 11 fließt der Ausgangsstrom $I_A$ zum Instrument 12.

Der Instrumentenschaltkreis 29 ist bei dem in Figur 4 dargestellten Instrument 12 komplexer ausgeführt als beim Ausführungsbeispiel gemäß Figur 3. Auch bei dem in Figur 4 dargestellten Instrument 12 ist die erste Elektrode 15 über den Koppelkondensator 31 mit dem ersten Instrumenteneingang 32 elektrisch verbunden. Die zweite Elektrode 16 ist über die Wicklungen eines Spartransformators 51 und einen dazu in Reihe geschalteten Reihenkondensator 52 mit der dritten Elektrode 16 sowie dem dritten Instrumenteneingang 34 elektrisch verbunden. Ein Mittenabgriff des Spartransformators 51 zwischen seinen beiden Wicklungen ist über eine Schalteinrichtung 53 mit dem ersten Instrumenteneingang 32 elektrisch verbunden. Die Schalteinrichtung 53 wird abhängig von einem Betätigungszustand einer Bedieneinheit 54 zwischen einem leitenden Zustand und einem sperrenden Zustand umgeschaltet. Die Bedieneinheit 54 kann einen oder mehrere manuell betätigbare Taster, Schalter oder andere Bedienelemente 55 aufweisen, die am Instrument 12 zugänglich angeordnet sind und von einer Bedienperson bedient beziehungsweise betätigt werden können.

Die Bedieneinheit 54 ist beispielsweise elektrisch mit dem zweiten Instrumenteneingang 33 verbunden, der über den zweiten Geräteausgang 36 mit der Steuereinrichtung 47 des Versorgungsgeräts 11 kommunikationsverbunden ist, so dass die Steuereinrichtung 47 abhängig vom Betätigungszustand der Bedieneinheit 54 das Netzteil 23 steuern kann. Dadurch kann beispielsweise die Ausgangsspannung $U_A$ eingeschaltet, ausgeschaltet oder deren Betrag beziehungsweise Amplitude verändert werden.

Die Versorgungsgeräte 11 gemäß der Figuren 3 und 4 können eine Bedienschnittstelle 59 aufweisen, mittels der eine Bedienperson einen Betriebsmodus des Versorgungsgeräts 11 einstellen kann, der durch einen Betriebsmodusparameter M gekennzeichnet ist (Figur 5). Über die Bedienschnittstelle können optional auch ein oder mehrere weitere Parameter vorgegeben oder eingestellt werden, beispielsweise eine elektrische Leistung, ein Crest-Faktor, eine Wellenform der Ausgangsspannung $U_A$, usw.

Bei allen Ausführungsbeispielen kann das Instrument 12 eine Bedieneinheit 54 aufweisen oder ohne Bedieneinheit ausgebildet sein. Bei allen Ausführungsbeispielen kann die Bedienung zusätzlich oder alternativ zu der Bedieneinheit 54 am Instrument 12 mittels der Bedienschnittstelle 59 am Versorgungsgerät 11 erfolgen, zu der auch separate über Kabel oder drahtlos mit dem Versorgungsgerät 11 kommunikationsverbundene Hand- und/oder Fußschalter, usw. gehören können.

Die im Zusammenhang mit den Ausführungsbeispielen gemäß der Figuren 3 und 4 erwähnte Sollwerteinstelleinrichtung 26 weist eine Ermittlungseinheit 60 sowie eine Korrektureinheit 61 auf. Die Ermittlungseinheit 60 ist dazu eingerichtet, abhängig von dem Betriebsmodusparameter M (also dem gewählten Betriebsmodus des Versorgungsgeräts 11) und einem von der Korrektureinheit 61 bereitgestellten Korrekturwert C, die Ausgangssollspannung $U_S$ zu ermitteln. Beispielsweise kann jedem Betriebsmodusparameter M eine Betriebsmodusspannung $U_M$ zugeordnet sein, die basierend auf dem Korrekturwert C verändert und insbesondere erhöht wird, wodurch eine korrigierte Betriebsmodusspannung $U_{MC}$ erhalten wird. Abhängig von dieser korrigierten Betriebsmodusspannung $U_{MC}$ kann dann die Ausgangssollspannung $U_S$ ermittelt und dem Netzteil 23 bereitgestellt werden.

Bei einer bevorzugten Ausführungsform entspricht der Betriebsmodusparameter M einer Betriebsmodusspannung $U_M$ und der Korrekturwert C einer Korrekturspannung $U_C$, die zur korrigierten Betriebsmodusspannung $U_{MC}$ addiert werden, wie es in Figur 6 dargestellt ist. Eine Sollwertausgabeeinheit 62 stellt dann die Ausgangssollspannung $U_S$ basierend auf der korrigierten Betriebsmodusspannung $U_{MC}$ ein.

Zur Ermittlung des Korrekturwertes C bzw. der Korrekturspannung $U_C$ wird der Korrektureinheit 61 der aktuelle Istwert des Ausgangsstromes $I_A$ übermittelt. Der Korrektureinheit 61 ist außerdem der Impedanzparameter ZI bekannt, der die Impedanz des Instruments 12 und insbesondere des Instrumentenschaltkreises 29 beschreibt. Anhand des Impedanzparameters ZI und dem Istwert des Ausgangsstromes $I_A$ kann dann der Korrekturwert C und beispielsgemäß die Korrekturspannung $U_C$ gemäß folgender Gleichung ermittelt werden:

$$U_C = Z \cdot I_A \ ,$$

wobei die Impedanz Z des Instruments aus dem Impedanzparameter ZI erhalten wird oder gleich dem Impedanzparameter ZI ist.

Über das Netzteil 23 mit dem Netzteilregler 25 wird dann die Ausgangsspannung $U_A$ entsprechend der Ausgangssollspannung $U_S$ geregelt. Hierzu kann die Ausgangsspannung $U_A$ über den Spannungssensor 27 erfasst und eine Differenzspannung $U_D$ zwischen der Ausgangssollspannung und dem Istwert der Ausgangsspannung $U_A$ berechnet und dann dem Netzteilregler 25 bereitgestellt werden. Der Netzteilregler 25 regelt die Ausgangsspannung $U_A$ mit dem Ziel, die bereitgestellte Differenzspannung $U_D$ zu minimieren.

Durch das Verändern und insbesondere Erhöhen der korrigierten Betriebsmodusspannung $U_{MC}$ gegenüber der Betriebsmodusspannung $U_M$ basierend auf dem Korrekturwert C bzw. der Korrekturspannung $U_C$ können Blindspannungen kompensiert werden, die durch den Instrumentenschaltkreis 29 und/oder parasitäre Effekte auftreten. Dadurch wird eine Abweichung zwischen der an einer der Elektroden 15, 16, 17 des Instruments 12 anliegenden Elektrodenspannung $U_E$ gegenüber der durch den Betriebsmodusparameter M definierten Betriebsmodusspannung $U_M$ reduziert oder eliminiert. Im Idealfall ist die Elektrodenspannung $U_E$ gleich der Betriebsmodusspannung $U_M$.

Die Korrektureinheit 61 kann beispielsweise eine Recheneinheit 65 und einen Speicher 66 aufweisen. Die Recheneinheit 65 kann ein geeigneter Schaltkreis zur Durchführung von mathematischen und/oder logischen Operationen sein, beispielsweise ein Mikrocomputer, ASIC oder dergleichen. Der Speicher 66 kann ein flüchtiger und/oder nicht flüchtiger Speicher sein. In dem Speicher 66 kann der Impedanzparameter ZI für das bzw. für jedes an das Versorgungsgerät 11 anschließbare Instrument 12 abgespeichert sein. In diesem Fall können das Versorgungsgerät 11 und das Instrument 12 dazu eingerichtet sein, das Instrument oder den Instrumenten zu erkennen. Beispielsweise kann das Instrument 12 einen Instrumentenspeicher 67 aufweisen, in dem eine Kennung K abgespeichert ist, die bei hergestellter elektrischer Verbindung mit dem Versorgungsgerät 11 ausgelesen und der Korrektureinheit 61 bereitgestellt werden kann. Anhand der Kennung K kann der zum Instrument 12 gehörende Impedanzparameter ZI ermittelt werden. Eine entsprechende Zuordnung in Form einer Tabelle oder dergleichen kann im Speicher 66 abgelegt sein.

Alternativ ist es auch möglich, im Instrumentenspeicher 67 den zum Instrument 12 gehörenden Impedanzparameter ZI abzuspeichern und auszulesen, wenn das Instrument 12 an das Versorgungsgerät 11 angeschlossen ist. Dann steht der Impedanzparameter ZI der Korrektureinheit 61 direkt zur Verfügung. In jedem Fall kann gemäß einer Funktion und insbesondere einer Multiplikation des Istwertes des Ausgangsstromes $I_A$ mit dem bereitgestellten oder ermittelten Impedanzparameter ZI die Korrekturspannung $U_C$ ermittelt werden. Der Istwert des Ausgangsstromes $I_A$ und der Impedanzparameter ZI können auch durch andere Funktionen miteinander verknüpft werden, um einen Korrekturwert C zu erhalten, beispielsweise einen Korrekturfaktor, mit dem die Betriebsmodusspannung $U_M$ multipliziert werden kann.

In Figur 7 ist ein beispielhafter zeitlicher Verlauf eines von der Elektrode 15, 16 in das zu behandelnde Gewebe G fließenden Elektrodenstromes $I_E$ sowie die Elektrodenspannung $U_E$ an dieser Elektrode veranschaulicht. Es ist zu erkennen, dass die Elektrodenspannung $U_E$ konstant bleibt, unabhängig vom Betrag des Elektrodenstromes $I_E$ während jedes an die Elektrode 15, 16 angelegten Impulses. Dieser Effekt ist darauf zurückzuführen, dass der Blindspannungsanteil aufgrund des Stromes, um den sich die Elektrodenspannung $U_E$ im nicht kompensierten Fall reduzieren würde, über den Korrekturwert C, bzw. die Korrekturspannung $U_C$ kompensiert wird. Die an der Elektrode 15, 16 anliegende Elektrodenspannung $U_E$ entspricht somit der gewünschten Betriebsmodusspannung $U_M$, die durch den gewählten Betriebsmodus (vorgegeben über den Betriebsmodusparameter M) an der Elektrode 15, 16 tatsächlich anliegen soll.

Bei einer Ausführungsform der Erfindung kann die Elektrodenspannung $U_E$ im phasenweise oder in einem oder mehreren Zeitintervallen ohne die vorstehend erläuterte Spannungskompensation erzeugt werden. Wenigstens ein Zeitintervall ohne Spannungskompensation der Elektrodenspannung $U_E$ kann mit wenigstens einem Zeitintervall mit Spannungskompensation der Elektrodenspannung $U_E$ zeitlich mittelbar oder unmittelbar aufeinanderfolgend kombiniert werden. Beispielsweise kann auf eine beliebige Anzahl N von Zeitintervallen mit Spannungskompensation der Elektrodenspannung $U_E$ eine beliebige Anzahl M von Zeitintervallen mit Spannungskompensation der Elektrodenspannung $U_E$ folgen oder umgekehrt. Dabei können die Anzahl N und die Anzahl M beliebige natürliche Zahlen (1, 2, 3, ...) sein. Dadurch lassen sich optional weitere vorteilhafte Effekte bei der Behandlung von biologischem Gewebe erzeugen.

Die Erfindung betrifft ein Versorgungsgerät 11 und ein Verfahren zur Versorgung eines elektromedizinischen Instruments 12 mit elektrischer Energie. Ein einstellbarer und/oder auswählbarer Betriebsmodusparameter M definiert eine Betriebsmodusspannung $U_M$, die einen Sollwert für eine Elektrodenspannung $U_E$ an einer Elektrode 15, 16 des elektromedizinischen Instruments 12 angibt. An einem Netzteilausgang 24 eines Netzteils 23 des Versorgungsgeräts 11 wird eine eingeprägte Ausgangsspannung $U_A$ entsprechend einer Ausgangssollspannung $U_S$ sowie ein variabler Ausgangsstrom $I_A$ für das elektromedizinische Instrument 12 bereitgestellt. Aufgrund von der Impedanz des Instruments 12 kann sich eine Blindspannung im elektrischen Pfad zu der Elektrode 15, 16 bilden, so dass die Elektrodenspannung $U_E$ nicht der gewünschten Betriebsmodusspannung $U_M$ entspricht. Deswegen wird die Betriebsmodusspannung basierend auf einem Korrekturwert C und insbesondere einer Korrekturspannung $U_C$ korrigiert und bildet eine korrigierte Betriebsmodusspannung $U_{MC}$, basierend auf der die Ausgangssollspannung $U_S$ für das Netzteil 23 vorgegeben wird. Der Korrekturwert C bzw. die Korrekturspannung $U_C$ werden abhängig vom Istwert des Ausgangsstromes $I_A$ sowie einem Impedanzparameter ZI ermittelt, der die Impedanz des Instruments 12 kennzeichnet.

Bezugszeichenliste:

| | |
|---|---|
| 10 | System |
| 11 | Versorgungsgerät |
| 12 | elektromedizinisches Instrument |
| 12a | bipolares Instrument |
| 12b | monopolares Instrument |
| 13 | Kabel |
| 14 | Stecker |
| 15 | erste Elektrode |
| 16 | zweite Elektrode |
| 17 | dritte Elektrode |
| 18 | Neutralelektrode |
| 19 | Elektrodenkabel |
| | |
| 23 | Netzteil |
| 24 | Netzteilausgang |
| 25 | Netzteilregler |
| 26 | Sollwerteinstelleinrichtung |
| 27 | Spannungssensor |
| 28 | Stromsensor |
| 29 | Instrumentenschaltkreis |
| 30 | Bauelement |
| 31 | Koppelkondensator |
| 32 | erster Instrumenteneingang |
| 33 | zweiter Instrumenteneingang |
| 34 | dritter Instrumenteneingang |
| 35 | erster Geräteausgang |
| 36 | zweiter Geräteausgang |
| 37 | dritter Geräteausgang |
| | |
| 41 | Transformator |
| 42 | Primärwicklung |
| 43 | Sekundärwicklung |
| 44 | Kondensator |
| 45 | gesteuerter Schalter |
| 46 | Ausgangsstufe |
| 47 | Steuereinrichtung |
| | |
| 51 | Spartransformator |
| 52 | Reihenkondensator |
| 53 | Schalteinrichtung |
| 54 | Bedieneinheit |
| 55 | Bedienelement |
| | |
| 59 | Bedienerschnittstelle |
| 60 | Ermittlungseinheit |
| 61 | Korrektureinheit |
| 62 | Sollwertausgabeeinheit |
| | |
| 65 | Recheneinheit |
| 66 | Speicher |
| 67 | Instrumentenspeicher |
| | |
| C | Korrekturwert |
| G | Gewebe |

$I_A$     Ausgangsstrom
$I_E$     Elektrodenstrom
K     Kennwert
M     Betriebsmodusparameter
$U_A$     Ausgangsspannung
$U_C$     Korrekturspannung
$U_D$     Differenzspannung
$U_E$     Elektrodenspannung
$U_{E1}$     erste Elektrodenspannung
$U_{E2}$     zweite Elektrodenspannung
$U_M$     Betriebsmodusspannung
$U_{MC}$     korrigierte Betriebsmodusspannung
$U_S$     Ausgangssollspannung
$U_V$     Versorgungsspannung
ZI     Impendanzwert

**Patentansprüche**

1. Versorgungsgerät (11), das zur Versorgung eines elektromedizinischen Instruments (12) mit elektrischer Energie eingerichtet ist, wobei das Versorgungsgerät (11) aufweist:

   - ein Netzteil (23), das dazu eingerichtet ist, an seinem Netzteilausgang (24) einen lastabhängigen Ausgangsstrom ($I_A$) und eine eingeprägte Ausgangsspannung ($U_A$) bereitzustellen, die einer Ausgangssollspannung ($U_S$) entspricht,
   - eine Sollwerteinstelleinrichtung (26), die eine Ermittlungseinheit (60) und eine Korrektureinheit (61) aufweist, wobei die Korrektureinheit (61) dazu eingerichtet ist, abhängig von einem aktuellen Istwert des Ausgangsstromes ($I_A$) und abhängig von mindestens einem Impedanzparameter (ZI), der eine elektrische Impedanz des zu versorgenden elektromedizinischen Instruments (12) charakterisiert, einen Korrekturwert (C) zu ermitteln, und wobei die Ermittlungseinheit (60) dazu eingerichtet ist, basierend auf einem eingestellten Betriebsmodusparameter (M) und dem Korrekturwert (C) die Ausgangssollspannung ($U_S$) zu ermitteln.

2. Versorgungsgerät nach Anspruch 1, wobei der Betriebsmodusparameter (M) eine Betriebsmodusspannung ($U_M$) ist und der Korrekturwert (C) eine Korrekturspannung ($U_C$) ist und wobei die Ermittlungseinheit (60) dazu eingerichtet ist, basierend auf der Betriebsmodusspannung ($U_M$) und der Korrekturspannung ($U_C$) eine korrigierte Betriebsmodusspannung ($U_{MC}$) zu ermitteln und einer Sollwertausgabeeinheit (62) zu übermitteln, die dazu eingerichtet ist, die Ausgangssollspannung ($U_S$) basierend auf der korrigierte Betriebsmodusspannung ($U_{MC}$) zu ermitteln und dem Netzteil (23) bereitzustellen.

3. Versorgungsgerät nach Anspruch 2, wobei die Ermittlungseinheit (60) dazu eingerichtet ist, die korrigierte Betriebsmodusspannung ($U_{MC}$) als eine Summe aus der Betriebsmodusspannung ($U_M$) und der Korrekturspannung ($U_C$) zu bilden.

4. Versorgungsgerät nach einem der vorhergehenden Ansprüche, wobei der Impedanzparameter (ZI) in der Korrektureinheit (61) unveränderbar oder veränderbar gespeichert ist.

5. Versorgungsgerät nach einem der vorhergehenden Ansprüche, wobei das Versorgungsgerät (11) dazu eingerichtet ist, einen Kennwert (K) eines an das Versorgungsgerät (11) angeschlossenen elektromedizinisches Instruments (12) zu ermitteln und der Korrektureinheit (61) bereitzustellen, die dazu eingerichtet ist, den Impedanzparameter (ZI) basierend auf dem Kennwert (K) zu ermitteln.

6. Versorgungsgerät nach Anspruch 5, wobei der Kennwert (K) den Impedanzparameter (ZI) aufweist oder der Impedanzparameter (ZI) ist.

7. Versorgungsgerät nach Anspruch 5, wobei der Kennwert (K) das individuelle elektromedizinische Instrument (12) oder den Instrumententyp beschreibt und die Korrektureinheit (61) dazu eingerichtet ist, den Kennwert (K) zu einem Impedanzparameter (ZI) zuzuordnen.

8. Versorgungsgerät nach einem der vorhergehenden Ansprüche, wobei die Korrektureinheit (61) dazu eingerichtet ist, den Impedanzparameter (ZI) abhängig von einem Betriebsmodus zu ermitteln.

9. Versorgungsgerät nach einem der vorhergehenden Ansprüche, wobei die Korrektureinheit (61) dazu eingerichtet ist, den Korrekturwert (C) und insbesondere die Korrekturspannung ($U_C$) mittels einer vorgegebenen Funktion zu berechnen.

10. Versorgungsgerät nach einem der vorhergehenden Ansprüche, wobei der wenigstens eine Impedanzparameter (ZI) abhängig ist von einer Frequenz des Ausgangsstromes ($I_A$) und/oder der Ausgangsspannung ($U_A$).

11. System (10) aufweisend ein elektromedizinisches Instrument (12), das an das Versorgungsgerät (11) nach einem der vorhergehenden Ansprüche angeschlossen ist.

12. System nach Anspruch 11, wobei das elektromedizinische Instrument (12) wenigstens eine Elektrode (15, 16, 17) und einen Instrumentenschaltkreis (29) aufweist, mit dem die Elektrode (15) oder zumindest eine der Elektroden (15, 16, 17) elektrisch verbunden ist.

13. System nach Anspruch 11, wobei der Instrumentenschaltkreis (29) ein kapazitives und/oder induktives Bauelement (30) aufweist.

14. System nach Anspruch 11 oder 12, wobei der Instrumentenschaltkreis (29) einen Koppelkondensator (31) aufweist, der mit der Elektrode (15) oder zumindest einer der Elektroden (15) elektrisch verbunden ist.

15. Verfahren zur Versorgung eines elektromedizinischen Instruments (12) mit elektrischer Energie mittels eines Versorgungsgerätes (11), wobei das Verfahren aufweist:

- Bereitstellen eines lastabhängigen Ausgangsstromes ($I_A$) und einer eingeprägten Ausgangsspannung ($U_A$) an einem Netzteilausgang (24) eines Netzteils (23) des Versorgungsgerätes (11) derart, dass die Ausgangsspannung ($U_A$) einer Ausgangssollspannung ($U_S$) entspricht,
- Ermitteln eines Korrekturwertes (C) abhängig von einem aktuellen Istwert des Ausgangsstromes ($I_A$) und abhängig von einem Impedanzparameter (ZI) zu ermitteln, der eine elektrische Impedanz des zu versorgenden elektromedizinischen Instruments (12) charakterisiert, und
- Ermitteln der Ausgangssollspannung ($U_S$) basierend auf einem eingestellten Betriebsmodusparameter (M) des Versorgungsgerätes (11) und dem Korrekturwert (C).

Fig. 1

Fig. 2

Fig. 3

**Fig. 4**

**Fig. 5**

Fig. 6

Fig. 7

Europäisches
Patentamt

European
Patent Office

Office européen
des brevets

# EUROPÄISCHER RECHERCHENBERICHT

Nummer der Anmeldung

EP 25 15 4540

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 4 458 300 A1 (ERBE ELEKTROMEDIZIN [DE]) 6. November 2024 (2024-11-06) | 1-3,5,6, 9-13,15 | INV. A61B18/12 |
| Y | * das ganze Dokument * | 14 | |
| A | | 4,8 | ADD. A61B18/00 |
| | ----- | | |
| X,D | DE 689 11 867 T2 (SMITHS INDUSTRIES PLC [GB]) 14. April 1994 (1994-04-14) | 1-5,7, 9-11,15 | |
| A | * das ganze Dokument * | 6,8 | |
| | ----- | | |
| Y | US 2007/118102 A1 (MIHORI TAKASHI [JP]) 24. Mai 2007 (2007-05-24) * Absätze [0017] - [0020]; Abbildung 1 * | 14 | |
| | ----- | | |
| A | DE 93 21 224 U1 (ERBE ELEKTROMEDIZIN [DE]) 5. September 1996 (1996-09-05) * das ganze Dokument * | 5-7 | |
| | ----- | | |
| A | EP 2 520 240 A1 (ERBE ELEKTROMEDIZIN [DE]) 7. November 2012 (2012-11-07) * Absätze [0012], [0028], [0031] - [0034]; Abbildungen 1-3 * | 5-7 | |
| | ----- | | RECHERCHIERTE SACHGEBIETE (IPC) |
| A | DE 10 2017 110473 A1 (WINTER & IBE OLYMPUS [DE]) 15. November 2018 (2018-11-15) * das ganze Dokument * | 5-7 | A61B |
| | ----- | | |

Der vorliegende Recherchenbericht wurde für alle Patentansprüche erstellt

| Recherchenort | Abschlußdatum der Recherche | Prüfer |
|---|---|---|
| Den Haag | 17. Juni 2025 | Friedrich, Franz |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
    ..........................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 25 15 4540

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-06-2025

| Im Recherchenbericht angeführtes Patentdokument | Datum der Veröffentlichung | Mitglied(er) der Patentfamilie | Datum der Veröffentlichung |
|---|---|---|---|
| EP 4458300 A1 | 06-11-2024 | CN 118892356 A | 05-11-2024 |
| | | EP 4458300 A1 | 06-11-2024 |
| | | JP 2024160955 A | 15-11-2024 |
| | | KR 20240161767 A | 12-11-2024 |
| | | US 2024366283 A1 | 07-11-2024 |
| DE 68911867 T2 | 14-04-1994 | AU 620459 B2 | 20-02-1992 |
| | | CA 2001637 A1 | 10-05-1990 |
| | | DE 68911867 T2 | 14-04-1994 |
| | | EP 0368532 A2 | 16-05-1990 |
| | | ES 2047684 T3 | 01-03-1994 |
| | | GB 2225656 A | 06-06-1990 |
| | | JP 2771642 B2 | 02-07-1998 |
| | | JP H02181208 A | 16-07-1990 |
| | | US 4961047 A | 02-10-1990 |
| US 2007118102 A1 | 24-05-2007 | JP 2007135914 A | 07-06-2007 |
| | | US 2007118102 A1 | 24-05-2007 |
| DE 9321224 U1 | 05-09-1996 | KEINE | |
| EP 2520240 A1 | 07-11-2012 | CN 102764153 A | 07-11-2012 |
| | | EP 2520240 A1 | 07-11-2012 |
| | | JP 6258247 B2 | 10-01-2018 |
| | | JP 2012232142 A | 29-11-2012 |
| | | JP 2015126925 A | 09-07-2015 |
| | | PL 2520240 T3 | 31-05-2017 |
| | | US 2012283733 A1 | 08-11-2012 |
| DE 102017110473 A1 | 15-11-2018 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102015212359 A1
- DE 68911867 T2